# EUROPEAN PATENT SPECIFICATION

(11) **EP 3 042 690 B1**
(45) Date of publication and mention of the grant of the patent: **14.04.2021**
(21) Application number: 14843133.1
(22) Date of filing: 03.09.2014
(51) Int. Cl.: A61M 37/00

(54) **MICRO-NEEDLE SHEET**
MIKRONADELFOLIE
FEUILLE À MICRO-AIGUILLES

(30) Priority: 06.09.2013 JP 2013185322
(43) Date of publication of application: 13.07.2016
(73) Proprietor: Hisamitsu Pharmaceutical Co., Inc., Tosu-shi, Saga 841-0017 (JP)
(72) Inventor: YAMAMOTO, Naoki, Tsukuba-shi Ibaraki 305-0856 (JP); OGURA, Makoto, Tsukuba-shi Ibaraki 305-0856 (JP)
(74) Representative: Grünecker Patent- und Rechtsanwälte PartG mbB
(86) International application number: PCT/JP2014/073195
(87) International publication number: WO 2015/033959

(56) References cited:
- EP-A1- 1 360 935
- WO-A1-2013/187392
- JP-A- 2007 501 070
- JP-A- 2007 521 090
- JP-A- 2007 535 388
- JP-A- 2009 501 066
- US-A1- 2005 245 845
- US-A1- 2007 293 816
- US-A1- 2008 125 743
- US-A1- 2010 130 940
- US-A1- 2011 034 860

## Description

### Technical Field

An embodiment of the present invention relates to a microneedle sheet used for assisting in administration of an active component by microneedles.

### Background Art

Microneedles for administrating active components through skin and devices including the microneedles are conventionally known.

For example, a rotatable microstructure apparatus disclosed in Patent Literature 1 below includes a curved substrate and a roller structure including a plurality of microelements affixed upon a first surface of the substrate. The microelements are of predetermined sizes and shapes so as to penetrate a stratum corneum layer of skin when the microstructure apparatus is placed upon the skin and rolled over the skin in a predetermined direction. Lancets for injecting into the skin, which rise upon bending a sheet, on which the lancets are provided, are known from US 2005/245845 A1 and EP 1,360,935 A1. Moreover, microneedle sheets with an array of protruding microneedles are known from US 2007/293816 A1 and US 2008/125743 A1.

### Citation List

### Patent Literature

[Patent Literature 1] Japanese Unexamined Patent Publication No. 2005-503210

### Summary of Invention

### Technical Problem

Unfortunately, in the microstructure apparatus disclosed in Patent Literature 1 above, the microelements are exposed on the roller and, therefore, the needles may touch or get caught in other objects (for example, the user's skin or clothes) before an active component is applied on the skin through the microneedles. It is therefore requested to ensure safety during the handling of microneedles.

### Solution to Problem

In order to solve the aforementioned problem, the present invention provides a microneedle sheet having the features defined in claim 1. Further preferred embodiments are defined in the dependent claims.

In such an aspect, the microneedles generally conform to the principal surface until the sheet is bent. This means that the tip ends of the microneedles do not protrude from the principal surface before the microneedles are applied on the skin. There is therefore no concern that the microneedles touch or get caught in other objects unless the microneedle sheet is applied on the skin. As a result, the safety during the handling of the microneedles can be ensured.

In addition, since at least some of the microneedles each have a region for exposing a tear formed in the skin, at least some of minute tears produced by insertion are not hidden by the microneedles but exposed. Even without removing the microneedles, an active component enters the skin through the tears, and the dose of the active component can be increased, accordingly.

### Advantageous Effects of Invention

According to an aspect of the present invention, safety during the handling of microneedles can be ensured.

### Brief Description of Drawings

FIG. 1 is a plan view of a microneedle sheet according to an embodiment.
FIG. 2 is a perspective view of an applicator according to an embodiment.
FIG. 3 is a side view corresponding to FIG. 2.
FIG. 4 is a side view showing the microneedle sheet mounted on the applicator.
FIG. 5 is a partial enlarged view of FIG. 4.
FIG. 6 is a diagram schematically showing a manner of insertion.
FIG. 7 is a diagram showing microneedles piercing the skin.
FIG. 8 is a diagram showing microneedles in Example and Comparative Example.
FIG. 9 is a graph showing the accumulated amount of permeation in Example and Comparative Example.
FIG. 10 is a diagram showing microneedles according to a modification.
FIG. 11 is a diagram showing microneedles according to another modification (said modification is not covered by the claims).

### Description of Embodiments

Embodiments of the present invention will be described in detail below with reference to the accompanying drawings. In the description of the drawings, the same or equivalent components are denoted with the same reference signs and an overlapping description will be omitted.

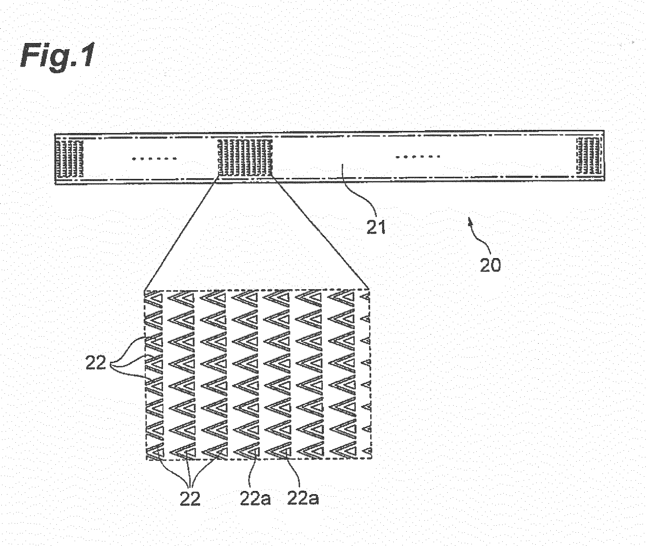

Referring to FIGS. 1 to 3, the structure of an applicator 10 and a microneedle sheet 20 according to an embodiment will be described. The microneedle sheet 20 is an instrument having a number of microneedles to be pierced the skin, and the applicator 10 is an assistance device for applying the microneedle sheet 20 on the skin.

First, the microneedle sheet 20 will be described. As shown in FIG. 1, the microneedle sheet 20 is shaped like a strip and has a plurality of microneedles 22 formed on a sheet generally along the principal surface 21 of the sheet. These microneedles 22 are arranged in alignment with each of the longitudinal direction and the width direction of the sheet. The tip ends of all of the microneedles 22 are oriented toward one end of the sheet (leftward in FIG 1) without exception.

As shown in FIG. 1, in the present embodiment, the outer shape of the microneedle 22 is approximately an isosceles triangle, and holes 22a are formed in some or all of the microneedles 22. The shape of the hole 22a is generally similar to the shape of the microneedle 22 itself and, more specifically, an isosceles triangle of which base is generally coincident with the root of the microneedle 22. Taking the hole 22a into consideration, therefore, the microneedle 22 can be said to be V-shaped. The shape, the position, and the size of the hole 22a are not limited to those in the example in FIG 1. For example, the shape of the hole 22a may be different from the outer shape of the microneedle 22, or the base of the hole 22a may be at a position shifted by a predetermined distance from the root toward the tip end of the microneedle 22. It should be noted that the hole 22a is formed so as to intersect the surface of skin in the needle inserted state.

The microneedle sheet 20 and the microneedles 22 are of any material. For example, the microneedle sheet 20 and the microneedles 22 may be fabricated from any one of stainless steel, polyethylene terephthalate (PET), other metals, other resins, biodegradable materials, ceramics, and bioabsorbable materials. Alternatively, the microneedle sheet 20 and the microneedles 22 may be fabricated from these materials in combination.

The microneedles 22 can be formed by etching. If the sheet is metallic, the microneedles 22 can be formed by etching the sheet by chemicals. If the sheet is non-metallic, the microneedles 22 can be formed by cutting the sheet by laser. The holes 22a can be formed by a similar method. In these cases, a gap is produced on the periphery of the microneedles 22. It is needless to say that the microneedles 22 can be formed by any technique other than etching. The microneedle sheet 20 can be produced readily and inexpensively because there is no need for raising the microneedles 22 from the principal surface 21 of the sheet in advance.

The microneedle sheet 20 may be of any size. Specifically, the lower limit of the thickness may be 1 µm, 5 µm, or 20 µm, and the upper limit of the thickness may be 1000 µm, 300 µm, 100 µm, or 50 µm. The lower limit of the length may be 0.1 cm or 1 cm, and the upper limit of the length may be 50 cm or 20 cm. The lower limit of the width may be 0.1 cm or 1 cm, and the upper limit of the width may be 60 cm or 30 cm. The lower limits of the length and the width of the microneedle sheet 20 are determined considering the dose of active components, and the upper limits of the length and the width are determined considering the size of the living body.

Parameters pertaining to the microneedles 22 may have any value. Specifically, the lower limit of the height of each needle may be 10 µm, 50 µm, or 100 µm, and the upper limit of the height may be 1000 µm, or 500 µm. The lower limit of the density of needles may be 0.05 needle/cm² or 1 needle/cm², and the upper limit of the density may be 10000 needles/cm² or 5000 needles/cm². The lower limit of the density is a value obtained in terms of the number of needles and the area with which 1 mg of an active component can be administered. The upper limit of the density is a threshold value in consideration of the shapes of the needles.

The proportion R of the region taken up by a hole 22a in one microneedle 22 may also be determined as desired. This proportion R is represented by R=Ab/Aa, where Aa is the area of the microneedle 22 (the area of the plane defined by the outer periphery and the root (base) of the microneedle), and Ab is the area of the region taken up by the hole 22a (the area of the plane defined by the outer periphery of the hole 22a). The lower limit of the proportion R may be 0.1 (that is, 10%), and the upper limit thereof may be 0.9 (that is, 90%) or may be 0.81 (that is, 81%).

An active component to be applied to skin may be prepared by the following procedures: coating the microneedle sheet 20 per se with an active component in advance; applying an active component on skin before inserting the microneedles 22 into the skin; and inserting the microneedles 22 into skin and thereafter applying an active component on the skin. If the microneedle sheet 20 is coated with an active component in advance, it is preferable to apply a coating liquid having a predetermined viscosity at a thickness as uniform as possible over the entire sheet. Such application can be easily done because the microneedles 22 conform to the principal surface 21. The coating may be carried out using the principles of screen printing or may be carried out by any other method. Since a hole 22a is formed in the microneedle 22, the microneedle 22 can be coated with more coating liquid than a microneedle without a hole 22a by the amount corresponding to the space of the hole 22a. The amount of an active component for coating is therefore increased, and the dose of the active component is increased, accordingly. An active component may be applied on both surfaces of the microneedle 22. In this case, the coating solution comes into contact with the skin at both surfaces of the microneedle 22 in the skin after insertion, so that the active component in the coating quickly dissolves and permeates into the skin. When a biodegradable sheet is used, an active component may be included in the sheet per se.

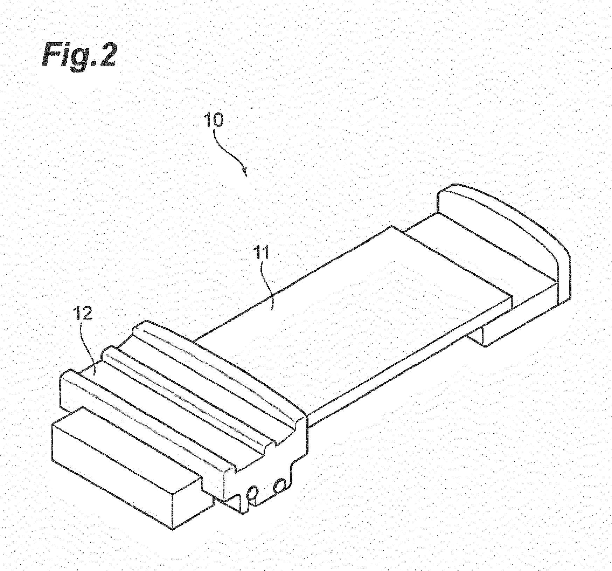

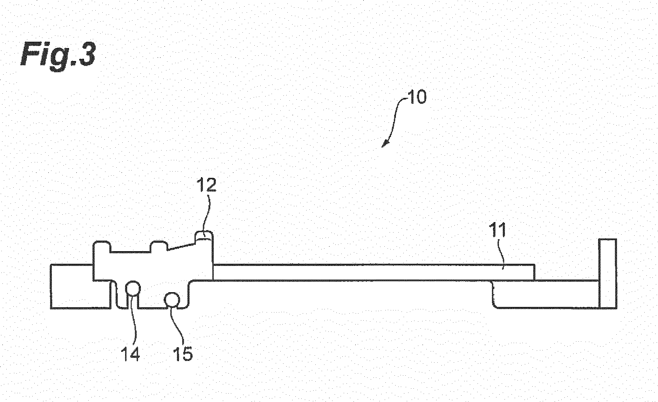

Referring now to FIGS. 2 and 3, the structure of the applicator 10 will be described. The applicator 10 has an elongated shape as a whole and includes a guide plate 11 extending in the longitudinal direction and a slider 12 provided on the guide plate 11. In the present embodiment, the left side, the right side, the upper side, and the lower side in FIG 3 (side view) are defined as the front side, the back side, the upper side, and the lower side of the applicator 10, respectively.

Examples of the material of the applicator 10 include plastics such as acrylics. The applicator 10, however, may be fabricated from any material, for example, using a metal or any other resin.

The size of the applicator 10 may be determined in accordance with the size of the microneedle sheet 20. For example, the width (the length in the direction orthogonal to the longitudinal direction) of the applicator 10 may be determined in accordance with the width of the microneedle sheet 20. The entire length (the length along the longitudinal direction) of the applicator 10 may be determined considering the length of the microneedle sheet 20 or the range of application of the microneedle sheet 20 to skin.

The guide plate 11 is an elongated plate-shaped member extending linearly and has a foot at its back end. In order to guide the microneedle sheet 20 to the lower surface of the guide plate 11, a hole (not shown) is formed on the back side of the guide plate 11.

The slider 12 is attached to the guide plate 11 so as to be able to move along the longitudinal direction of the guide plate 11. Considering the easiness of slide operation, protrusions and depressions are formed on the upper surface of the slider 12. This slider 12 includes two elongated cylindrical members extending along the width direction. The first cylindrical member 14 is provided at a position close to the lower surface of the guide plate 11. The second cylindrical member 15 is provided at the lower portion of the slider 12 (at a position where it nearly abuts on the surface of skin during use of the applicator 10) and to the rear of the first cylindrical member 14. The diameters of these two cylindrical members are set in accordance with the thickness of the microneedle sheet 20 and the length (height) of the microneedles 22 and are each, for example, 1 to 4 mm.

In the inside of the slider 12, a guide path is formed for allowing the microneedle sheet 20 to pass through from the vicinity of the lower surface of the guide plate 11 to the surface of skin. Specifically, this guide path extends from the back end of the slider 12 to the first cylindrical member 14 generally horizontally, folds back downward at the first cylindrical member 14, then extends to the second cylindrical member 15, and folds back downward again at the second cylindrical member 15 to reach the surface of skin. In the present embodiment, the direction in which the microneedle sheet 20 is moved is reversed (changed by approximately 180 degrees) at the first cylindrical member 14, because the position of the lower end of the first cylindrical member 14 and the position of the upper end of the second cylindrical member 15 are almost the same in the height direction. Their heights, however, may be different from each other. For example, if the distance between the first cylindrical member 14 and the second cylindrical member 15 in the height direction is increased, the direction in which the microneedle sheet 20 is moved is changed by less than 180 degrees.

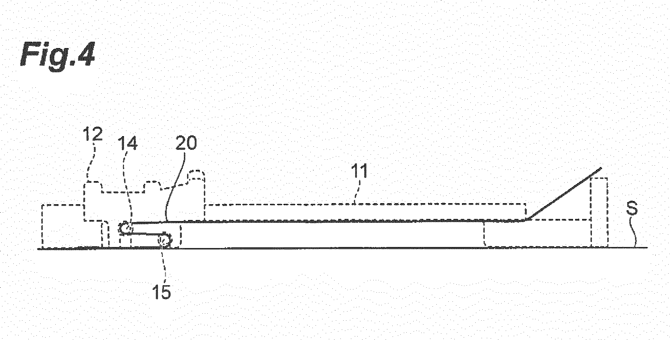

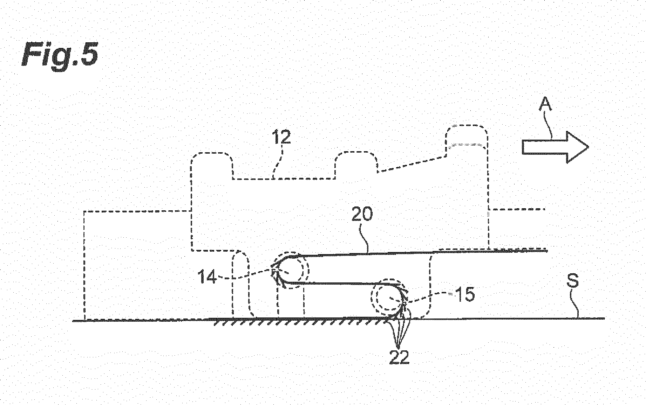

Referring now to FIGS. 4 to 6, the usage of the applicator 10 and the microneedle sheet 20 will be described. In FIGS. 4 and 5, the microneedle sheet 20 is denoted by a solid line and the applicator 10 is denoted by a broken line in order to facilitate understanding as to how the microneedle sheet 20 is set in the applicator 10.

First, the user sets the microneedle sheet 20 in the applicator 10. Specifically, the user passes the microneedle sheet 20 through the hole at the back side of the guide plate 11 and further through the guide path in the slider 12 and then takes out one end of the microneedle sheet 20 to the underside of the second cylindrical member 15. The user then puts the slider 12 on the vicinity of the front end of the guide plate 11 and places the applicator 10 on skin S such that one end of the microneedle sheet 20 taken out from the guide path faces the front of the applicator 10.

Through a series of these operations, the microneedle sheet 20 is set as shown in FIG. 4. That is, the microneedle sheet 20 passed through the hole at the back end of the guide plate 11 is guided along the lower surface of the guide plate 11 to the slider 12 and is bent into the shape of an S by the two cylindrical members 14 and 15 in the slider 12 to reach the skin S.

In doing so, the user fixes the front end of the microneedle sheet 20 on the skin S with a finger, an adhesive tape, or other means so that the microneedle sheet 20 is not displaced on the skin due to the slide operation described later. Alternatively, the front end of the microneedle sheet 20 may be provided with adhesive for fixing.

After placing the applicator 10 and the microneedle sheet 20 at a place where the active component is to be applied, the user moves the slider 12 toward the back end of the guide plate 11 (in the direction denoted by the arrow A in FIG. 5). This slide operation allows the microneedle sheet 20 to be guided to the second cylindrical member 15 corresponding to the bending portion, and the portion of the microneedle sheet 20 that reaches the second cylindrical member 15 is bent at that position. As shown in FIG. 5, the microneedles 22 located at the bent portion are then raised from the principal surface 21 of the sheet, and the raised microneedles 22 pierce the skin S.

A row of the microneedles 22 along the width direction of the microneedle sheet 20 are raised at a time at the second cylindrical member 15. The angle between the raised microneedle 22 and the principal surface 21 is greater than 0 degrees and less than 180 degrees, as a matter of course. Although the microneedles 22 are also raised temporarily when passing through the first cylindrical member 14, this does not interfere with the passage of the microneedles 22 through the second cylindrical member 15.

The user moves the slider 12 by a desired distance, so that a plurality of microneedles 22 in the range of the distance pierce the skin. The user therefore can administer a desired amount of the active component by adjusting the application area of the microneedle sheet 20.

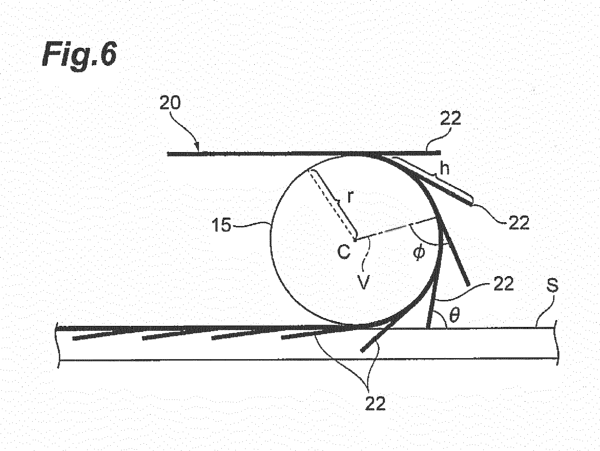

As shown in FIG. 6, the angle of insertion θ (the angle between the microneedle 22 and the skin S) formed when the microneedle 22 raised from the principal surface 21 pierces the skin is also greater than 0 degrees and less than 180 degrees. The lower limit of the angle of insertion may be 20 degrees, 34 degrees, or 40 degrees, and the upper limit of the angle may be 160 degrees, 140 degrees, or 100 degrees.

The value r in FIG 6 denotes the radius of curvature of the tip end of the second cylindrical member 15. The maximum angle φ formed between the microneedle 22 raised from the principal surface 21 by folding of the microneedle sheet 20 and an imaginary line V extending from the center of curvature C to the root of the microneedle 22 is greater than 90 degrees. For example, the maximum angle φ may be in a range of 95 to 130 degrees or may be in a range of 95 to 120 degrees. Setting the maximum angle φ to be greater than 90 degrees in this manner increases the length of the portion of the microneedle that pierces the skin, thereby increasing the permeation of the active component into the skin.

The ratio (h/r) of the needle length h to the radius of curvature r may be set to be greater than 0.20 to ensure that the microneedle 22 pierces the skin S.

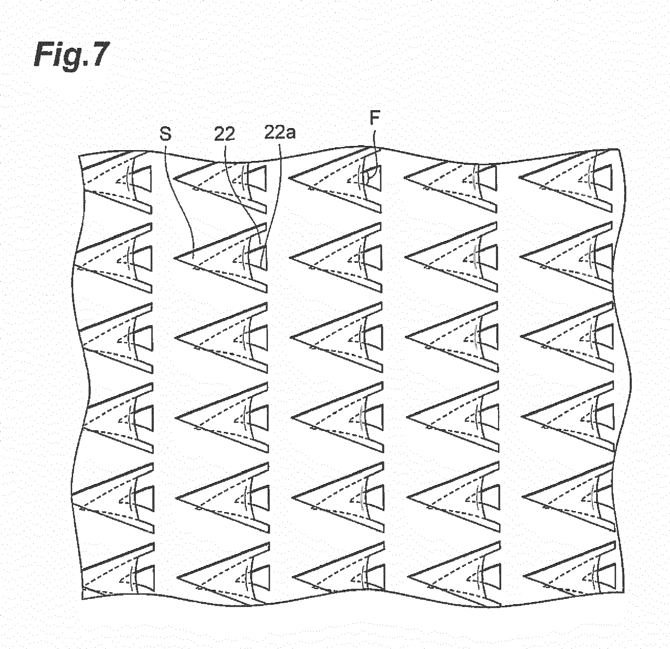

FIG. 7 schematically shows the microneedles 22 piercing the skin S. The entire microneedle 22 may enter the skin S completely or the microneedle 22 may partially enter. When the microneedle 22 partially enters the skin S, the root side of the microneedle 22 remains exposed on the skin S. Accordingly, the hole 22a also entirely or partially enters the skin S, and the remaining portion thereof is positioned on the skin S even after insertion. The hole 22a thus intersects the surface of the skin S in the needle inserted state. A tear F in the skin S corresponding to the intersection is not blocked by the microneedle 22. Therefore, when an active component is applied on the skin with the microneedle sheet 20 kept applied on the skin even after insertion, the active component is allowed to penetrate into the skin through the tear F. As shown in FIGS. 1 and 7, it is advantageous to form the hole 22a so as to extend from the root toward the tip end of the microneedle in that the active component can be penetrated into the skin, because the tear F can be exposed more reliably in the needle inserted state.

The microneedle sheet 20 is suitable for combination with application of a chemical solution with high flowability. This is because a minute tear F made by insertion is exposed, the chemical solution then penetrates into the skin through the tear F, and, as a result, the active component can directly permeate into the skin.

The user moves the applicator 10 on the skin by a desired distance to allow a plurality of microneedles 22 in the range of the distance to pierce the skin. The user therefore can administer a desired amount of the active component by adjusting the application area of the microneedle sheet 20.

As described above, according to the present embodiment, the microneedles 22 extend generally along the principal surface 21 of the sheet until the cylindrical members 14 and 15 of the applicator 10 bend the microneedle sheet 20. This means that the tip ends of the microneedles 22 do not protrude from the principal surface 21. There is therefore no concern that the microneedles 22 touch or get caught in other objects (for example, the user's skin or clothes) unless the applicator 10 is used. As a result, the safety during the handling of the microneedles 22 can be ensured. For example, the user can safely carry out storage and conveyance of the microneedle sheet 20 or make preparations immediately before use.

Since the microneedle sheet 20 is thin and flexible, the sheet 20 can be put on the skin in conformity with the shape of a living body, and, as a result, the active component can be efficiently administered.

In addition, since at least some of the microneedles 22 have holes 22a for exposing tears F formed in the skin, at least some of the minute tears F produced by insertion are not hidden by the microneedle 22 but exposed. Therefore, without removing the microneedles 22, the active component enters the skin through the tears F, and the dose of the active component can be increased, accordingly.

The applicator 10 inserts the microneedles 22 into skin by raising the microneedles 22 and pushing the raised microneedles 22 into skin, rather than giving impact to the microneedle sheet 20. Thus, an active component can be administered to the subject without causing a sense of fear.

### [Example]

Although the present invention will now be specifically described based on examples, the present invention is not intended to be limited thereto.

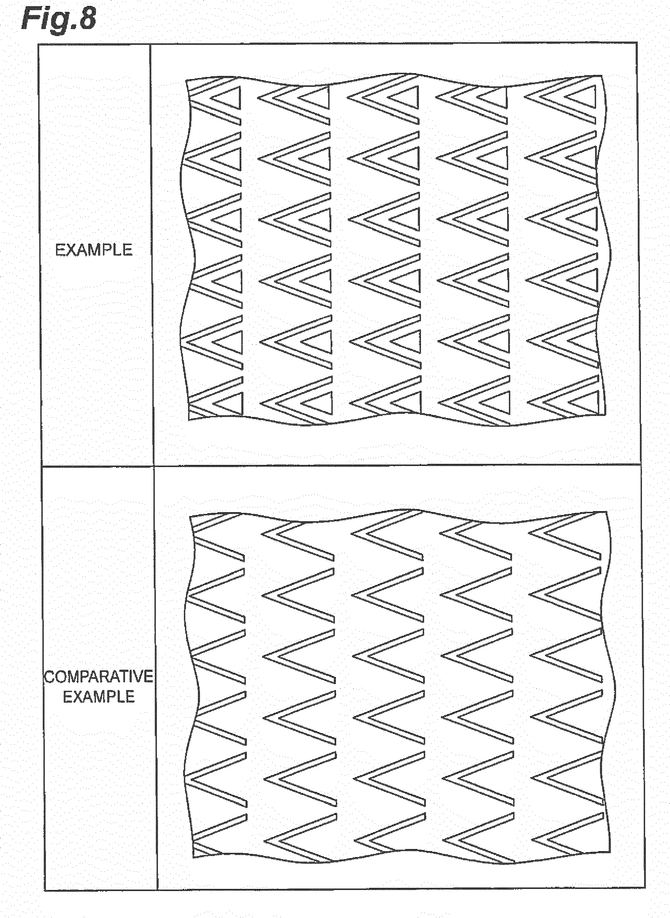

Human skin was used, and the amount of a chemical permeated into the skin was determined when two kinds of microneedle sheets were applied. As shown in FIG. 8, in Example, V-shaped microneedles similar to the microneedles 22 in the foregoing embodiment are formed, whereas in Comparative Example, triangular microneedles without holes are formed. The size of the microneedle sheet is the same in Example and Comparative Example, namely, the length is 20 mm and the width is 10 mm. In both of Example and Comparative Example, each individual microneedle has a height of about 500 µm and a width of about 400 µm at the root. In Example, the length of the hole along the height direction of the microneedle is about 300 µm, and the base of the hole at the root of the microneedle is about 250 µm.

The method of applying the microneedle sheet to human skin is the same in Example and Comparative Example, specifically, as follows. First, the microneedle sheet was arranged in a casing having a cylindrical thin rod (hereinafter referred to as "cylindrical rod"), and the microneedle sheet was folded using the cylindrical rod to raise the microneedles. The cylindrical rod was then moved along the upper surface of human skin to allow the microneedles to pierce the human skin. With the microneedle sheet kept applied on the human skin, the human skin was set in a flow-through cell. An aqueous solution of galantamine hydrobromide was then applied on the microneedle sheet inserted in the human skin, and the amount of galantamine hydrobromide permeated into the skin was determined over 24 hours since the application. Sampling was conducted every two hours using phosphate buffered saline (PBS) as a receiver solution.

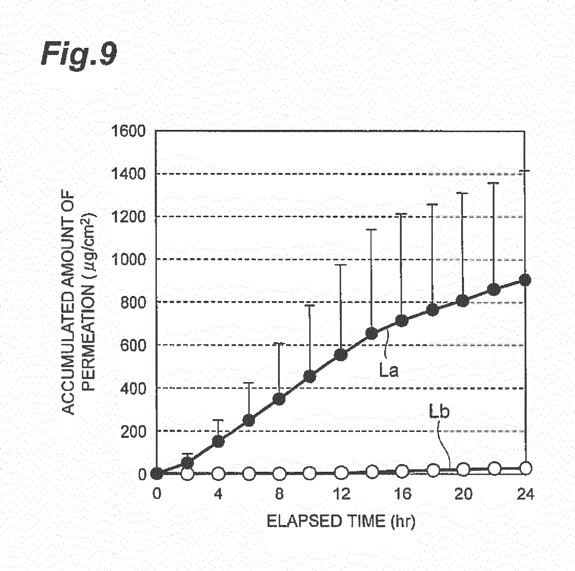

The accumulated amount of permeation over time since the application is shown in FIG. 9. In this graph, the vertical axis represents the accumulated amount of permeation (µg/cm²), and the horizontal axis represents the elapsed time (hours). The line La represents Example, and the line Lb represents Comparative Example. It is understood from this graph that when an active component is applied on the microneedle sheet with the microneedle sheet kept applied, the use of such microneedles that expose tears of skin in the needle inserted state is effective in terms of administration of an active component.

The present invention has been described above based on the embodiment. The present invention, however, is not intended to be limited to the foregoing embodiment.

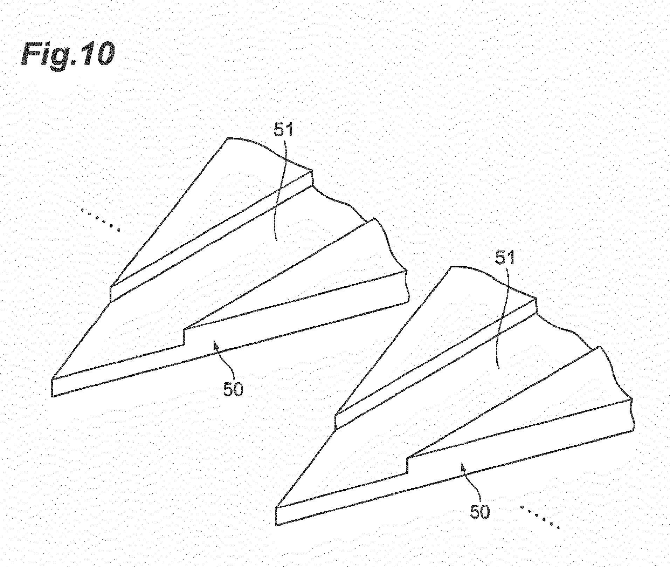

The shape of the microneedle is not intended to be limited to that of the foregoing embodiment. For example, microneedles 50 as shown in FIG 10 may be employed. The microneedle 50 has a groove 51 extending in the height direction. The start point, the end point, and the length of the groove 51 can be set as desired as long as the groove 51 intersects the surface of the skin in the needle inserted state. In the example in FIG. 10, the groove 51 is formed from the root to the tip end of the microneedle 50, on the surface of the microneedle 50 along the principal surface of the microneedle sheet. In place of the groove 51 or in addition to the groove 51, a groove may be formed on the surface of the microneedle 50 orthogonal to the principal surface of the microneedle sheet.

When the microneedle 50 pierces the skin, that portion of the tear in the skin made by the insertion which corresponds to the groove 51 is not blocked by the microneedle 50 but exposed, so that the active component can enter the skin through the exposed portion. It is advantageous to form the groove 51 so as to extend from the root toward the tip end of the microneedle in that the active component is penetrated into the skin, because the tear can be exposed more reliably in the needle inserted state.

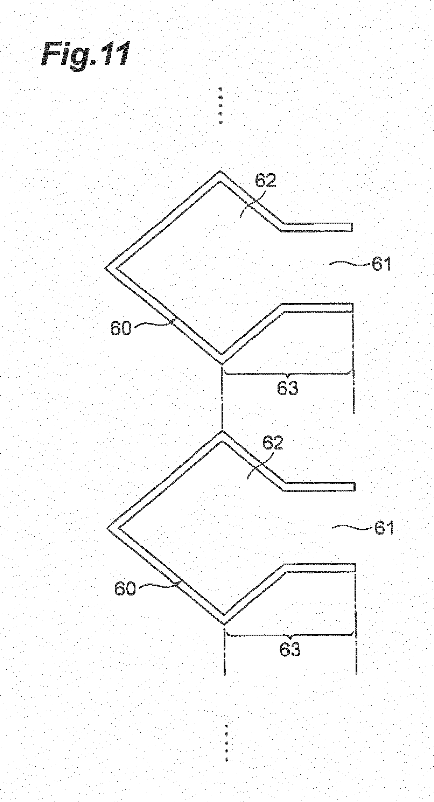

Alternatively, microneedles 60 as shown in FIG 11 may be employed. The microneedle 60 includes a rectangular root portion 61 and an approximately rhombic tip end portion 62 provided in front of the root portion 61 and is shaped like an arrowhead as a whole. The reason why the tip end portion 62 is formed into a rhombic shape is to facilitate removal of the microneedle 60 from the skin after insertion. The width of the microneedle 60 is largest approximately at the center of the tip end portion 62, and the width of the microneedle 60 in a section 63 from the center to the root is smaller than the largest width. The length of the section 63 corresponding to the narrow width portion is set such that the section 63 intersects the surface of the skin in the needle inserted state.

When the microneedle 60 pierces the skin, a part of the tear in the skin made by the insertion is not blocked by the microneedle 60 but exposed, so that the active component can enter the skin through the exposed portion.

As described above, the region for exposing the tear formed in the skin may be a hole, a groove, or a narrow width portion.

The shape and the structure of the applicator are not intended to be limited as long as the applicator has a component that bends the microneedle sheet. For example, the applicator may be a simple cylindrical rod or may include any given mechanical, electrical, or electronic structure or control means.

The shape of the microneedle sheet is not limited to a strip-like shape but may be, for example, a rectangle having nearly equal length and width or may be a circle or an ellipse.

The microneedle sheet 20 may be used singly, and the use of an applicator is not essential.

The microneedle sheet according to the present invention can be combined with electric (iontophoresis), pressure, magnetic-field, ultrasonic (sonophoresis), or other transdermal drug delivery techniques. A combination of the microneedle sheet with those other techniques can further increase the amount of chemical absorption.

### Reference Signs List

10 ... applicator, 11 ... guide plate, 12 ... slider, 14 ... first cylindrical member, 15 ... second cylindrical member, 20 ... microneedle sheet, 21 ... principal surface, 22 ... microneedle, 22a ... hole, 50 ... microneedle, 51 ... groove, 60 ... microneedle, 61 ... root portion, 62 ... tip end portion, 63 ... section corresponding to narrow width portion.

## Claims

1. A microneedle sheet (20) comprising a plurality of microneedles (22) formed on a sheet generally along a principal surface (21) of the sheet, wherein
the plurality of microneedles (22) are arranged in alignment with each of a longitudinal direction and a width direction of the sheet, and tip ends of the plurality of microneedles (22) are oriented toward one end of the sheet,
outer shapes of the plurality of microneedles (22) are approximately an isosceles triangle,
heights of the plurality of microneedles (22) are 10 µm to 1000 µm,
the microneedles (22) are configured to rise from the principal surface (21) when the sheet is bent, and the raised microneedles (22) pierce skin (S), and
at least some of the microneedles (22) each have a hole (22a) configured to intersect a surface of the skin (S) in a needle inserted state so as to expose a tear (F) formed in the skin (S) in the needle inserted state.

2. The microneedle sheet (20) according to claim 1, wherein the hole (22a) extends from a root toward a tip end of each of the microneedles (22).

3. The microneedle sheet (20) according to claim 1 or 2, wherein an angle of insertion of the raised microneedle (22) to the skin (S) is equal to or greater than 34 degrees and less than 180 degrees.

4. The microneedle sheet (20) according to any one of claims 1 to 3, wherein a maximum angle formed between the microneedle (22) raised from the principal surface (21) and an imaginary line (V) extending from a center of curvature (C) of the sheet to a root of the microneedle (22) is greater than 90 degrees.

5. The microneedle sheet (20) according claim 4, wherein the maximum angle is 95 to 130 degrees.

## Patentansprüche

1. Mikronadelbogen (20), umfassend eine Vielzahl von Mikronadeln (22), die auf einem Bogen im Allgemeinen entlang einer Hauptoberfläche (21) des Bogens ausgebildet sind, wobei
die Vielzahl von Mikronadeln (22) in Ausrichtung mit jeder von einer Längsrichtung und einer Breitenrichtung des Bogens angeordnet sind, und Spitzenenden der Vielzahl von Mikronadeln (22) zu einem Ende des Blattes hin ausgerichtet sind,
äußere Formen der Vielzahl von Mikronadeln (22) ungefähr ein gleichschenkliges Dreieck sind,
die Höhe der Vielzahl von Mikronadeln (22) 10 µm bis 1000 µm beträgt,
die Mikronadeln (22) so konfiguriert sind, dass sie sich von der Hauptoberfläche (21) erheben, wenn der Bogen gebogen wird, und die erhobenen Mikronadeln (22) die Haut (S) durchstechen, und
zumindest einige der Mikronadeln (22) jeweils ein Loch (22a) aufweisen, das so konfiguriert ist, dass es eine Oberfläche der Haut (S) in einem nadeleingeführten Zustand schneidet, um einen Riss (F) freizulegen, der in der Haut (S) in nadeleingeführten Zustand gebildet ist.

2. Mikronadelbogen (20) nach Anspruch 1, wobei sich das Loch (22a) von einer Wurzel zu einem Spitzenende jeder der Mikronadeln (22) erstreckt.

3. Das Mikronadelbogen (20) nach Anspruch 1 oder 2, wobei ein Einführungswinkel der angehobenen Mikronadel (22) zur Haut (S) gleich oder größer als 34 Grad und kleiner als 180 Grad ist.

4. Mikronadelbogen (20) nach einem der Ansprüche 1 bis 3, wobei ein maximaler Winkel, der zwischen der von der Hauptoberfläche (21) abgehobenen Mikronadel (22) und einer imaginären Linie (V) gebildet wird, die sich von einem Krümmungsmittelpunkt (C) des Bogens zu einer Wurzel der Mikronadel (22) erstreckt, größer als 90 Grad ist.

5. Mikronadelbogen (20) nach Anspruch 4, wobei der maximale Winkel 95 bis 130 Grad beträgt.

## Revendications

1. Feuille à micro-aiguilles (20) comprenant une pluralité de micro-aiguilles (22) formées sur une feuille généralement le long d'une surface principale (21) de la feuille,
la pluralité des micro-aiguilles (22) étant disposées en alignement avec chacun d'un sens longitudinal et d'un sens de la largeur de la feuille, et les extrémités de pointe de la pluralité des micro-aiguilles (22) étant orientées vers une extrémité de la feuille,
des formes externes de la pluralité des micro-aiguilles (22) étant approximativement un triangle isocèle,
les hauteurs de la pluralité des micro-aiguilles (22) étant de 10 µm à 1 000 µm,
les micro-aiguilles (22) étant conçues pour s'élever depuis la surface principale (21) lorsque la feuille est pliée, et les micro-aiguilles (22) élevées percent la peau (S), et
au moins certaines des micro-aiguilles (22) ayant chacune un trou (22a) conçu pour entrer en intersection avec une surface de la peau (S) sous un état inséré d'aiguille afin d'exposer une déchirure (F) formée dans la peau (S) sous l'état inséré d'aiguille.

2. Feuille à micro-aiguilles (20) selon la revendication 1, le trou (22a) s'étendant depuis une racine vers une extrémité de pointe de chacune des micro-aiguilles (22).

3. Feuille à micro-aiguilles (20) selon la revendication 1 ou 2, un angle d'insertion de la micro-aiguille (22) élevée par rapport à la peau (S) étant supérieur ou égal à 34 degrés et inférieur à 180 degrés.

4. Feuille à micro-aiguilles (20) selon l'une quelconque des revendications 1 à 3, un angle maximal formé entre la micro-aiguille (22) élevée depuis la surface principale (21) et une ligne imaginaire (V) s'étendant depuis un centre de courbure (C) de la feuille vers une racine de la micro-aiguille (22) étant supérieur à 90 degrés.

5. Feuille à micro-aiguilles (20) selon la revendication 4, l'angle maximal étant de 95 à 130 degrés.
